# EUROPEAN PATENT SPECIFICATION

(11) **EP 0 790 035 B1**
(45) Date of publication and mention of the grant of the patent: **13.11.2002**
(21) Application number: 97300997.0
(22) Date of filing: 17.02.1997
(51) Int. Cl.: A61B 5/15

(54) **Improvements relating to blood sampling devices**
Verbesserungen für Blutentnahmegeräte
Améliorations apportées aux dispositifs de prélèvement sanguin

(30) Priority: 17.02.1996 GB 9603311
(43) Date of publication of application: 20.08.1997
(73) Proprietor: OWEN MUMFORD LIMITED, Woodstock, Oxford OX20 1TU (GB)
(72) Inventor: Marshall, Jeremy, Jericho, Oxford OX2 6DD (GB)
(74) Representative: James, Michael John Gwynne

(56) References cited:
- EP-A- 0 061 102
- WO-A-93/21974
- WO-A-96/02189

## Description

This invention relates to blood sampling devices, and in particular to a lancet for pricking the skin to draw a small drop of blood for analysis. Such prickers are widely used by diabetics, for example, who need to know their sugar level. However, there are many other applications.

There is an obvious need to make the sharp tip of the lancet safe after use. There have been various proposals for having the lancet as part of a cheap, disposable device which, after a single use, can be thrown away with the lancet automatically retracted inside the body and thus made safe. These are very handy, but in the long run it is more expensive to use them than to have a permanent firing mechanism and to load it when required with a lancet. But then the lancet must be made safe after removal.

The usual form of lancet has a steel needle encased in an elongate plastics body. A cap is moulded integrally with this body and initially the tip of the needle is embedded in it. But the connection between the cap and the body is weak and the cap can be pulled free to expose the tip. After use, the cap may be used to cover the needle tip again, and it may be re-oriented to fit over the forward end of the body.

The needle tends to be smooth and the plastics material of the body does not have much grip on it. It can happen that, when breaking the cap free, the cap is squeezed or angled with respect to the body sufficiently to pull the needle forwardly by its tip with respect to the body. There is then a lancet with a needle tip extending too far.

There is also a trend towards making the needle thinner, to achieve an even sharper point, and therefore a less noticeable prick. But the needle also serves to give some strength to the lancet as a whole, providing a reinforcing rod along its axis. If it is too thin, the lancet is able to bend rather easily, and this is unsatisfactory.

These problems have largely been answered by the lancet described in WO96/02189 which comprises a needle, an elongate body of moulded plastics material encasing most of the needle, and a breakaway cap integrally moulded with the body and initially encasing the tip of the needle. This is exposed at the forward end of the body when the cap is removed, and the needle is longitudinally movable rearwardly within the body to retract the needle tip after use.

Thus, instead of trying to place the cap back over the end of the lancet after use, the needle is pushed backwards in relation to the body until the tip is flush with or recessed into the forward end. This pushing may be done using the original cap, which may be adapted for the operation. For example, the leading end of the body may be recessed, and the cap may have a projection which can enter the recess when pushing the tip of the needle.

However, there is still uncertainty that the tip will always be retracted sufficiently. It is the aim of this invention to provide an even safer lancet.

According to the present invention there is provided a lancet for a skin pricker comprising a needle with a tip and an elongate body of moulded plastics material encasing most of the needle, which is movable rearwardly with respect to the body to retract the needle tip within the body after use, the needle tip being initially encased by a breakaway cap integrally moulded with the body at its forward end to be exposed when the cap is removed, the mutual engagement of the needle and body being such that the needle cannot move forwards from its initial position relative to the body, and the needle and body remaining effectively integral and moving together during the act of skin pricking, characterised in that the body has a forward and rearward part joined by a bridge which acts as a spring normally holding the parts distanced but which is compressible to allow the parts to approach, the hold on the needle of the rearward part being greater than that of the forward part whereby, when the needle is pushed rearwardly with a pusher also acting on the forward part, the latter closes up towards the rearward part and the needle is retracted, and when the pusher is removed the rearward part holds the needle while the forward part is sprung forward by the bridge to shield the needle tip.

Conveniently the bridge is provided by an aperture diametrally through the body, and this may be an enlargement of one of the apertures which are formed by elements which hold the needle while the body is moulded around it. Preferably the aperture is generally elliptical or lozenge shaped in cross section, the major axis of that section being transverse to the length of the body whereby in the normal, relaxed position, the ellipse or lozenge has a significant minor axis, but when the parts are pressed together this axis is reduced while the major axis slightly lengthens.

The difference in hold may be achieved by different frictional grips, the rearward part being substantially longer than the forward part and thus having a much greater surface area in contact with the needle.

Alternatively the hold on the needle of the rearward part may be a membrane at the rear end of the body against which the rear end of the needle bears during skin pricking, but which can be broken when the needle is urged rearwardly by a substantial force.

A further option is for the hold on the needle of the rearward part to be a throat at the rear end of the body in which the rear end of the needle is trapped during skin pricking, but through which that rear end of the needle can be urged by a substantial force.

Conveniently the cap is adapted to serve as an implement for pushing the tip of needle, after use, back into the body. The forward end of the body may be recessed and the cap may have a projection which can enter the recess when pushing the tip of the needle.

Preferably, the rear end of the body is adapted to shield the rear end of the needle when the needle is retracted.

The impediment to forward movement of the needle from its initial position relative to the body may be provided by a forwardly facing shoulder where the needle reduces in cross section to a leading length portion projecting from the body and terminating in the tip. The needle will generally be cylindrical with a flat formed over the leading length portion to make the reduced cross section.

For a better understanding of the invention, one embodiment will now be described, by way of example, with reference to the accompanying drawings, in which:
Figure 1 is a side view of a lancet prior to use,
Figure 2 is a rear end view of the lancet,
Figure 3 is a side view of the lancet with its needle being retracted after use, and
Figure 4 is a side view of the lancet after retraction and ready to discard.

The lancet has an elongate body 1 of moulded plastics material. It resembles the spool of a spool valve, having alternating large and small diameter sections. The three rearmost larger diameter portions have ribs to co-operate with the interior of the firing device and stop the body 1 rotating. Co-axially encased within the body 1 there is a needle 2 which projects in a sharp tip 3 at the forward end. This tip is initially encased in a cap 4 which is in the form of a disc with a central domed stud 5 on one face. The cap is integrally moulded with the body 1 and connects to it by a neck 6 which will be weak enough to be sheared off from the end surface 7 by a twisting action. The forward end of the body has a transverse recess 8 in which the surface 7 forms the base.

At the other end, the body has a recess 9, initially empty but into which the rear end of the needle 2 projects when retracted.

The needle 2 is cylindrical over most of its length, but at its forward end a flat 10 is formed which extends back from the extremity of the tip to a radial, semicircular shoulder 11.

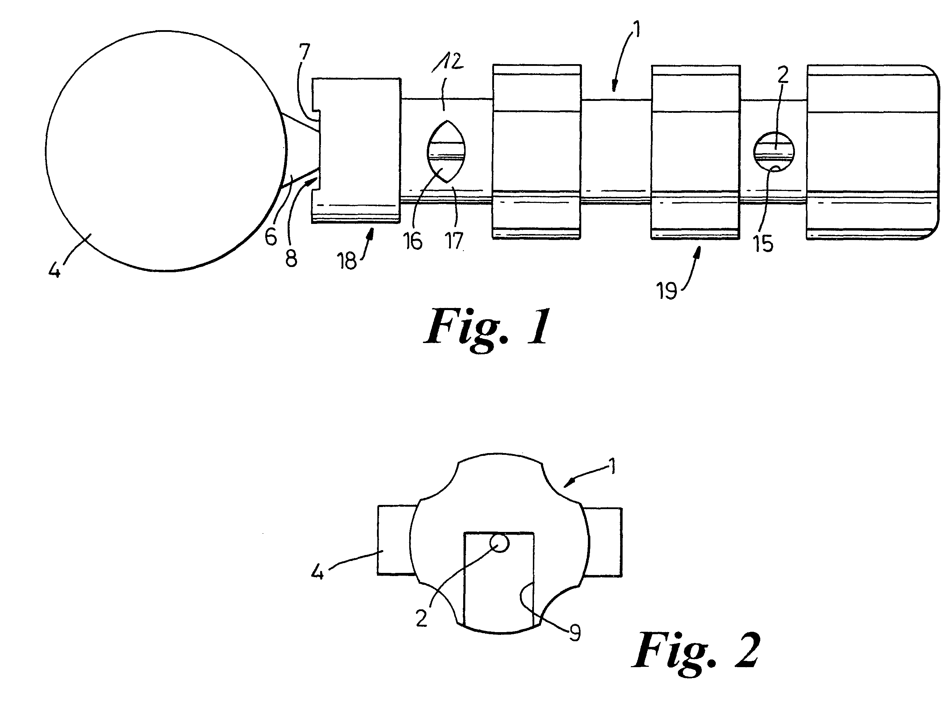

Thus far, the construction is very similar to that described in WO96/02189. However, there is a difference in the leading small diameter portion 12 of the body 1. Previously, this was recessed on one side and, in practice, had a diametrically opposed aperture on the other side as a result of the means which held the needle while the body 1 was moulded. There was a similar recess and aperture at the rear end. But these apertures were small and circular in cross section and the body remained substantially rigid. In this embodiment, there are still those recesses 13 and 14 and (disregarding the needle) the rear one 13 opens into a small circular aperture 15 as before. But the forward recess 14 opens into an enlarged aperture 16 whose cross section is substantially elliptical or lozenge shaped with the major axis transverse to the length of the body 1. This creates a narrow bridge 17 at each side dividing the body into a forward part 18 and a rearward part 19. In the normal, relaxed state as shown in Figure 1 the minor axis of the ellipse or lozenge is about two thirds the length of the major axis.

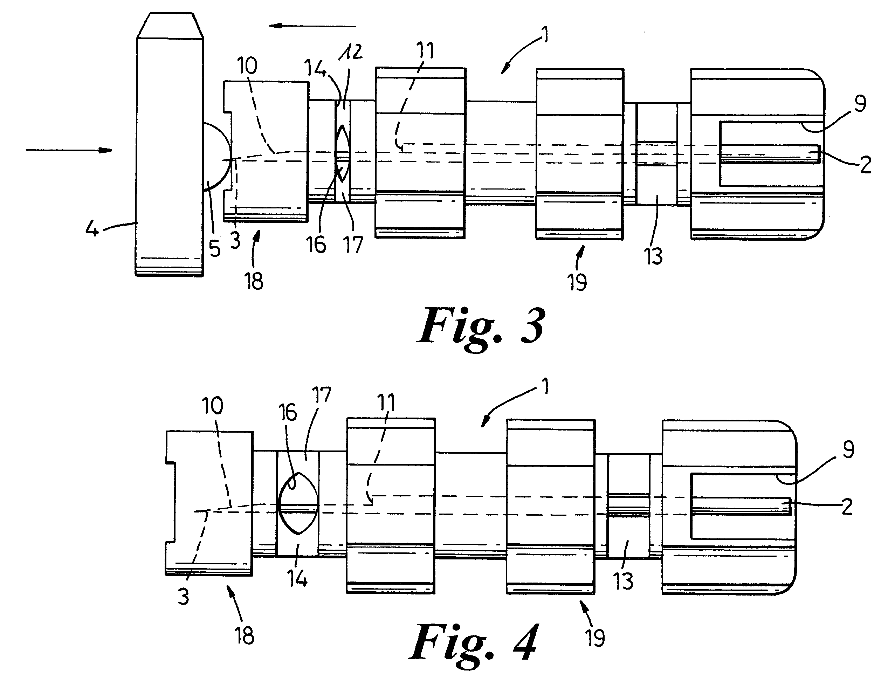

For use, the cap 4 is twisted off and the lancet is fired in the usual way. Afterwards, the cap 4 may be used to press the needle to a retracted position, using the stud 5 as shown in Figure 3. As it is pressed against the needle tip 3, that will embed itself slightly, but the needle will be shifted rearwardly until the stud 5 meets the surface 7. Continued pressure, with the user holding the rearward part 19, forces the forward part 18 back and causes the aperture 16 to contract along its minor axis and expand slightly along its major axis as shown in Figure 3. Meanwhile, the rear end of the needle projects into the recess 9.

The cap 4 is then removed, and the bridges 17, being of resilient plastics material, act as springs pushing the forward part 18 of the body 1 away from the rearward part 19 so that the body 1 resumes its original configuration. But a much greater length of needle 2 is within the rearward part 19 than the forward part 18, and the needle 2 is therefore held by the frictional grip of the rearward part 19 while the forward part 18 slides forwards relative to the tip 3, which is left concealed as shown in Figure 4.

Instead of relying just on friction, as mentioned above there could be a membrane initially across the rear end of the needle which remains entire when pricking the skin, but which is broken when the needle is forced back after use. Alternatively, there might be a throat which the rear end of the needle only penetrates when forced back after use.

## Claims

1. A lancet for a skin pricker comprising a needle (2) with a tip (3) and an elongate body (1) of moulded plastics material encasing most of the needle (2), which is movable rearwardly with respect to the body (1) to retract the needle tip (3) within the body (1) after use, the needle tip being initially encased by a breakaway cap (4) integrally moulded with the body (1) at its forward end to be exposed when the cap (4) is removed, the mutual engagement of the needle (2) and body (1) being such that the needle (2) cannot move forwards from its initial position relative to the body (1), and the needle (2) and body (1) remaining effectively integral and moving together during the act of skin pricking, **characterised in that** the body (1) has a forward (18) and rearward (19) part joined by a bridge (17) which acts as a spring normally holding the parts (18, 19) distanced but which is compressible to allow the parts (18, 19) to approach, the hold on the needle (2) of the rearward part (19) being greater than that of the forward part (18) whereby, when the needle (2) is pushed rearwardly with a pusher also acting on the forward part (18), the latter closes up towards the rearward part (19) and the needle (2) is retracted, and when the pusher is removed the rearward part (19) holds the needle (2) while the forward part (18) is sprung forward by the bridge (17) to shield the needle tip (3).

2. A lancet as claimed in Claim 1, **characterised in that** the bridge (17) is provided by an aperture (16) diametrally through the body (1).

3. A lancet as claimed in Claim 2, **characterised in that** the diametral aperture (16) is an enlargement of one of the apertures which are formed by elements which hold the needle (2) while the body (1) is moulded around it.

4. A lancet as claimed in Claim 2 or 3, wherein the aperture (16) is generally elliptical or lozenge shaped in cross section, the major axis of that section being transverse to the length of the body (1) whereby in the normal, relaxed position, the ellipse or lozenge has a significant minor axis, but when the parts are pressed together this axis is reduced while the major axis slightly lengthens.

5. A lancet as claimed in any preceding Claim, **characterised in that** the difference in hold is achieved by different frictional grips, the rearward part (19) being substantially longer than the forward part (18) and thus having a much greater surface area in contact with the needle (2).

6. A lancet as claimed in any one of Claims 1 to 4, **characterised in that** the hold on the needle (2) of the rearward part (19) is a membrane at the rear end of the body (1) against which the rear end of the needle (2) bears during skin pricking, but which can be broken when the needle (2) is urged rearwardly by a substantial force.

7. A lancet as claimed in any one of Claims 1 to 4, **characterised in that** the hold on the needle (2) of the rearward part (19) is a throat at the rear end of the body in which the rear end of the needle (2) is trapped during skin pricking, but through which that rear end of the needle (2) can be urged by a substantial force.

8. A lancet as claimed in any preceding Claim, **characterised in that** the cap (4) is adapted to serve as an implement for pushing the tip (3) of the needle (2), after use, back into the body (1).

9. A lancet as claimed in Claim 8, **characterised in that** the forward end of the body (1) is recessed and the cap (4) has a projection (5) which can enter the recess (8) when pushing the tip (3) of the needle (2).

10. A lancet as claimed in any preceding Claim, **characterised in that** the rear end of the body (1) is adapted to shield the rear end of the needle (2) when the needle (2) is retracted.

11. A lancet as claimed in any preceding Claim, **characterised in that** the impediment to forward movement of the needle (2) from its initial position relative to the body (1) is a forwardly facing shoulder (11) where the needle (2) reduces in cross section to a leading length portion projecting from the body and terminating in the tip (3).

12. A lancet as claimed in Claim 11, **characterised in that** the needle (2) is generally cylindrical with a flat (10) formed over the leading length portion to make the reduced cross section.

## Patentansprüche

1. Lanzette für eine Hautnadel mit einer Nadel (2) mit einer Spitze (3) und einem Längskörper (1) aus geformten Kunststoffwerkstoff, welcher den größten Teil der Nadel (2) umgibt, welche bezüglich des Körpers (1) rückwärts bewegbar ist, um nach Gebrauch die Nadelspitze (3) in den Körper (1) zurückzuziehen, wobei die Nadelspitze vor Gebrauch durch eine abbrechbare Kappe (4), welche einstückig mit dem Körper (1) an dessen vorderem Ende geformt ist, umschlossen ist, wobei die Nadelspitze frei liegt, wenn die Kappe (4) entfernt ist, wobei der gegenseitige Eingriff zwischen Nadel (2) und Körper (1) derart ausgebildet ist, daß sich die Nadel (2) von ihrer Ausgangsposition relativ zum Körper (1) nicht vorwärts bewegen kann und Nadel (2) und Körper (1) während des Einstechens in die Haut sich einstückig und zusammen bewegen,
**dadurch gekennzeichnet,**
**daß** der Körper (1) ein vorderes Teil (18) und ein hinteres Teil (19) aufweist, welche über eine Brücke (17) miteinander verbunden sind, die als Feder wirkt, welche die Teile (18, 19) normalerweise beabstandet hält, welche jedoch zusammendrückbar ist, um eine Annäherung der Teile (18, 19) zueinander zu erlauben, wobei eine Halterung der Nadel am hinteren Teil (19) größer ist als am vorderen Teil (19), wodurch sich der vordere Teil (18) dem hinteren Teil (19) nähert und die Nadel (2) eingezogen ist, wenn die Nadel (2) mit einer Schubvorrichtung, die auch auf den vorderen Teil (18) wirkt, zurück gezogen wird, und wobei der hintere Teil (19) die Nadel (2) hält während der vordere Teil (18) von der Brücke federnd vorwärts gedrückt wird, um die Nadelspitze (3) zu umgeben, wenn die Schubvorrichtung entfernt ist.

2. Lanzette nach Anspruch 1, **dadurch gekennzeichnet, daß** die Brücke (17) eine diametral durch den Körper (1) verlaufende Öffnung (16) aufweist.

3. Lanzette nach Anspruch 2, **dadurch gekennzeichnet, daß** die diametrale Öffnung (16) eine Vergrößerung einer der Öffnungen ist, die durch Elemente ausgebildet werden, die die Nadel (2) halten, während der Körper (1) darum herum geformt wird.

4. Lanzette nach Anspruch 2 oder 3, **dadurch gekennzeichnet, daß** die Öffnung (16) im Querschnitt im wesentlichen elliptisch oder rautenförmig ausgebildet ist, wobei eine Hauptachse in diesem Schnitt transversal zur Länge des Körpers (1) angeordnet ist, wodurch in der normalen, entspannten Position die Ellipse oder Raute eine signifikante Nebenachse aufweist, aber wenn die Teile zueinander gedrückt werden, diese Achse reduziert ist, während die Hauptachse leicht verlängert ist.

5. Lanzette nach einem der vorhergehenden Ansprüche, **dadurch gekennzeichnet, daß** die Differenz in der Halterung durch unterschiedliche Reibeingriffe erzielt wird, wobei der hintere Teil (19) im wesentlichen länger ist als der vordere Teil (18) und dadurch eine wesentlich größere Berührungsoberfläche mit der Nadel (2) aufweist.

6. Lanzette nach einem der Ansprüche 1 bis 4, **dadurch gekennzeichnet, daß** die Halterung der Nadel (2) am hinteren Teil (19) eine Membran am hinteren Ende des Körpers (1) ist, an der das hintere Ende der Nadel (2) während des Einstechens in die Haut anschlägt, welche jedoch bricht, wenn die Nadel (2) durch eine entsprechende Kraft rückwärts gedrückt wird.

7. Lanzette nach einem der Ansprüche 1 bis 4, **dadurch gekennzeichnet, daß** die Halterung der Nadel (2) im hinteren Teil (19) des Körpers ausgebildeter Hals ist, in dem das hintere Ende der Nadel (2) während des Einstechens in die Haut gefangen ist, wobei jedoch das hintere Ende der Nadel (2) durch diesen Hals mittels einer entsprechenden Kraft hindurch gedrückt werden kann.

8. Lanzette nach einem der vorhergehenden Ansprüche, **dadurch gekennzeichnet, daß** die Kappe (4) derart ausgebildet ist, daß diese als Mittel zum Eindrücken der Spitze (3) der Nadel (2) nach Gebrauch zurück in den Körper (1) hinein dient.

9. Lanzette nach Anspruch 8, **dadurch gekennzeichnet, daß** das vordere Ende des Körpers (1) eine Ausnehmung und die Kappe (4) eine Erhebung (5) aufweist, die in die Ausnehmung (8) eingreift, wenn die Spitze (3) der Nadel (2) eingedrückt wird.

10. Lanzette nach einem der vorhergehenden Ansprüche, **dadurch gekennzeichnet, daß** das hintere Ende des Körpers (1) zum schützenden Umgeben des hinteren Endes der eingezogen Nadel (2) ausgebildet ist.

11. Lanzette nach einem der vorhergehenden Ansprüche, **dadurch gekennzeichnet, daß** zum Verhindern einer Vorwärtsbewegung der Nadel (2) von ihrer Anfangsposition relativ zum Körper (1) eine in Vorwärtsrichtung ausgerichtete Schulter (11) ausgebildet ist, wobei sich ein Querschnitt der Nadel (2) in Richtung zu einem führenden Längsabschnitt verengt, der sich von dem Körper erhebt und in der Spitze (3) endet.

12. Lanzette nach Anspruch 11, **dadurch gekennzeichnet, daß** die Nadel (2) zylinderförmig mit einer Abflachung (10) am führenden Längsabschnitt ausgebildet ist, um den reduzierten Querschnitt auszubilden.

## Revendications

1. Lancette pour un dispositif à piquer la peau, comprenant une aiguille (2) ayant une pointe (3) et un corps allongé (1) de matière plastique moulée enfermant la majeure partie de l'aiguille (2), qui est déplaçable vers l'arrière par rapport au corps (1) pour rétracter la pointe (3) de l'aiguille à l'intérieur du corps (1) après utilisation, la pointe de l'aiguille étant initialement enfermée par un capuchon cassable (4) moulé d'un seul tenant avec le corps (1) à son extrémité avant pour être exposée lorsque le capuchon (4) est enlevé, l'engagement mutuel de l'aiguille (2) et du corps (1) étant tel que l'aiguille (2) ne peut pas se déplacer vers l'avant à partir de sa position initiale par rapport au corps (1), et l'aiguille (2) et le corps (1) restant effectivement solidaires et se déplaçant ensemble pendant l'action de piquage de la peau, **caractérisée par le fait que** le corps (1) a une partie avant (18) et une partie arrière (19) réunies par un pont (17) qui joue le rôle de ressort maintenant normalement les parties (18, 19) éloignées, mais qui est compressible pour permettre aux parties (18, 19) de s'approcher, le maintien sur l'aiguille (2) de la partie arrière (19) étant supérieur à celui de la partie avant (18), ce par quoi, lorsque l'aiguille (2) est poussée vers l'arrière avec un poussoir agissant également sur la partie avant (18), cette dernière se ferme vers la partie arrière (19) et l'aiguille (2) est rétractée, et lorsque le poussoir est retiré, la partie arrière (19) maintient l'aiguille (2) alors que la partie avant (18) est projetée vers l'avant par le pont (17) pour protéger la pointe (3) de l'aiguille.

2. Lancette selon la revendication 1, **caractérisée par le fait que** le pont (17) est doté d'une ouverture (16) diamétralement à travers le corps (1).

3. Lancette selon la revendication 2, **caractérisée par le fait que** l'ouverture diamétrale (16) est un agrandissement de l'une des ouvertures qui sont formées par des éléments qui maintiennent l'aiguille (2) alors que le corps (1) est moulé autour d'elle.

4. Lancette selon l'une des revendications 2 ou 3, dans laquelle l'ouverture (16) est généralement elliptique ou en forme de losange en section transversale, l'axe majeur de cette section étant transversal à la longueur du corps (1), ce par quoi, dans la position relaxée, normale, l'ellipse ou le losange a un petit axe significatif, mais, lorsque les parties sont comprimées ensemble, cet axe est réduit alors que le grand axe s'allonge légèrement.

5. Lancette selon l'une quelconque des revendications précédentes, **caractérisée par le fait que** la différence de maintien est obtenue par des saisies par frottement différentes, la partie arrière (19) étant sensiblement plus longue que la partie avant (18) et de ce fait ayant une aire de surface bien plus grande en contact avec l'aiguille (2).

6. Lancette selon l'une quelconque des revendications 1 à 4, **caractérisée par le fait que** le maintien sur l'aiguille (2) de la partie arrière (19) est une membrane à l'extrémité arrière du corps (1) contre laquelle l'extrémité arrière de l'aiguille (2) porte pendant le piquage de la peau, mais qui peut être rompu lorsque l'aiguille (2) est sollicitée vers l'arrière par une force substantielle.

7. Lancette selon l'une quelconque des revendications 1 à 4, **caractérisée par le fait que** le maintien sur l'aiguille (2) de la partie arrière (19) est une gorge à l'extrémité arrière du corps dans laquelle l'extrémité arrière de l'aiguille (2) est piégée pendant le piquage de la peau, mais à travers laquelle cette extrémité arrière de l'aiguille (2) peut être sollicitée par une force substantielle.

8. Lancette selon l'une quelconque des revendications précédentes, **caractérisée par le fait que** le capuchon (4) est adapté pour servir d'outil pour pousser la pointe (3) de l'aiguille (2), après utilisation, en retour dans le corps (1).

9. Lancette selon la revendication 8, **caractérisée par le fait que** l'extrémité avant du corps (1) est évidée et le capuchon (4) a une projection (5) qui peut entrer dans la cavité (8) lors de la poussée de la pointe (3) de l'aiguille (2).

10. Lancette selon l'une quelconque des revendications précédentes, **caractérisée par le fait que** l'extrémité arrière du corps (1) est adaptée pour protéger l'extrémité arrière de l'aiguille (2) lorsque l'aiguille (2) est rétractée.

11. Lancette selon l'une quelconque des revendications précédentes, **caractérisée par le fait que** l'obstacle au mouvement vers l'avant de l'aiguille (2) à partir de sa position initiale par rapport au corps (1) est un épaulement (11) tourné vers l'avant où l'aiguille (2) se réduit en section transversale à une partie de longueur d'attaque se projetant à partir du corps et se terminant dans la pointe (3).

12. Lancette selon la revendication 11, **caractérisée par le fait que** l'aiguille (2) est généralement cylindrique avec un méplat (10) formé sur la partie de longueur d'attaque pour réaliser la section transversale réduite.
